(19) Europäisches Patentamt
European Patent Office
Office européen des brevets

(11) **EP 2 123 224 A1**

(12) **EUROPEAN PATENT APPLICATION**
published in accordance with Art. 153(4) EPC

(43) Date of publication:
**25.11.2009 Bulletin 2009/48**

(51) Int Cl.:
**A61B 8/08** *(2006.01)*

(21) Application number: **07859898.4**

(22) Date of filing: **20.12.2007**

(86) International application number:
**PCT/JP2007/074550**

(87) International publication number:
**WO 2008/075740 (26.06.2008 Gazette 2008/26)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **20.12.2006 JP 2006342777**

(71) Applicant: **Hitachi Medical Corporation Chiyoda-ku Tokyo 101-0021 (JP)**

(72) Inventors:
- **ARAI, Osamu Tokyo 101-0021 (JP)**
- **MATSUMURA, Takeshi Tokyo 101-0021 (JP)**

(74) Representative: **Erny, Tobias MERH-IP Matias Erny Reichl Hoffmann Paul-Heyse-Strasse 29 80336 München (DE)**

(54) **ULTRASONOGRAPHIC DEVICE**

(57) An ultrasonic diagnostic apparatus is **characterized by** including a displacement/strain calculation unit 12 which obtains a strain distribution of a body site on a scan plane when pressed by an ultrasonic probe 1 and a non-pressed image creation unit 5 which corrects an ultrasonic image on the basis of the strain distribution calculated by the displacement/strain calculation unit and generates a corrected ultrasonic image in a non-pressed state or a pressed image creation unit 40 which generates a corrected reference image obtained by adding, to the reference image, a strain equivalent to one in the ultrasonic image on the basis of the strain distribution obtained by the displacement/strain calculation unit 12, in order to accurately perform comparative observation of an ultrasonic image and a reference image captured by a medical diagnostic apparatus other than the ultrasonic diagnostic apparatus.

FIG.7(A)

FIG.7(B)

**Description**

Technical Field

The present invention relates to an ultrasonic diagnostic apparatus and, more particularly, to a technique for pressing an ultrasonic probe against the body surface of an object and capturing an image.

Background Art

An ultrasonic diagnostic apparatus which is an example of an image diagnostic apparatus is easy to handle and is capable of noninvasively observing an arbitrary section in real time. Ultrasonic diagnostic apparatuses are thus very often used for diagnosis.

However, in ultrasonic diagnosis, an ultrasonic probe pressed against the body surface of an object and transmits and receives an ultrasonic wave in order to improve measurement sensitivity. Accordingly, a compressive force applied by the ultrasonic probe causes a body site in the object, such as an organ, to deform, and an ultrasonic image with strain is obtained.

The process of measuring, e.g., the distance to, the area of, and the volume of each site of a living body from an ultrasonic image and using measurement results for diagnosis has been proposed. A strain in an ultrasonic image, however, may adversely affect the accuracy of the measurement.

An ultrasonic image is generally inferior in image quality to a tomogram image captured by X-ray CT equipment or MRI equipment. For this reason, the process of improving the reliability of diagnosis by comprehensively performing diagnosis while using a CT image or an MR image as a reference image captured by an image diagnostic apparatus other than an ultrasonic diagnostic apparatus, such as X-ray CT equipment or MRT equipment, and comparing an ultrasonic image with the reference image has been proposed (see, e.g., Patent Document 1). According to the process, a tomogram image at the same section as a scan plane ot an ultrasonic image is extracted from multi-slice image data (hereinafter referred to as volume image data) of a CT image or an MR image and is rendered as a reference image on a display screen.

However, a reference image such as an MRI image or a CT image is captured without pressure on an object. Accordingly, the shape of a body site such as an organ in an ultrasonic image with strain may not coincide with that of the body site in a reference image, and the reliability of diagnosis by comparative observation may be damaged.

For example, although strain in a living-body tissue noticeably appears in an ultrasonic image which is a captured image of a soft site such as a mammary gland due to pressure applied by a probe, a reference image has no such strain.

Patent Document 1: WO2004/098414 A1

Disclosure of the Invention

The present invention has as its object to correct strain in an ultrasonic image with the strain which is obtained by pressing an ultrasonic probe against a body surface of an object and capturing an image or correct a reference image such that the reference image can be comparatively observed with the ultrasonic image.

In order to achieve the above-described object, a first aspect of the present invention is an ultrasonic diagnostic apparatus characterized by comprising an ultrasonic probe which is pressed against a body surface of an object and transmits and receives an ultrasonic wave to and from the object, ultrasonic image generation means for forming an ultrasonic image on a scan plane of the ultrasonic probe on the basis of RF signal frame data of a reflected echo signal received via the ultrasonic probe, and display means for displaying the ultrasonic image on a screen and is **characterized in that** strain calculation means for obtaining a strain distribution of a body site on the scan plane when pressed by the ultrasonic probe, on the basis of a pair of the RF signal frame data which are obtained at different measurement times and corrected ultrasonic image generation means for generating a corrected ultrasonic image in a non-pressed state in which no pressure is applied to the body site, on the basis of the strain distribution obtained by the strain calculation means are provided, and the display means displays the corrected ultrasonic image on the screen.

That is, as for an ultrasonic image, the ultrasonic probe is pressed against the body surface of the object and transmits and receives an ultrasonic wave, and an ultrasonic image in which a body site such as an organ in an object is deformed or strained by a compressive force applied by the ultrasonic probe is generated. Accordingly, an error occurs when the distance to, the area of, and the like of each body site is measured.

For this reason, according to the first aspect of the present intention, the strain distribution of the body site on the scan plane when pressed by the ultrasonic probe is obtained, the ultrasonic image is corrected on the basis of the obtained strain distribution to remove strain, and the corrected ultrasonic image in the non-pressed state in which no pressure is applied to the body site is generated. It is thus possible to improve the accuracy of measuring the distance to, the area of, the volume of, and the like of each body site on the basis of the ultrasonic image.

In this case, the strain calculation means can be configured to obtain a strain distribution of a region-of-interest which is set in the ultrasonic image displayed on the screen. The corrected ultrasonic image generation means can be configured to perform enlargement correction on the ultrasonic image on the basis of the strain distribution obtained by the strain calculation means such that the region-of-interest has a uniform distribution of strain and generate the corrected ultrasonic image.

In addition to the first aspect, the ultrasonic diagnostic apparatus can be configured to comprise storage means for storing volume image data other than an ultrasonic image captured by an image diagnostic apparatus in advance and reference image generation means for extracting tomogram image data corresponding to the ultrasonic image from the volume image data stored in the storage means and reconstructing a reference image and such that the display means displays the corrected ultrasonic image on a same screen as the reference image.

With this configuration, the corrected ultrasonic image in the non-pressed state is displayed on the same screen as the reference image, and the shape of a body site such as an organ in the corrected ultrasonic image and that of the body site in the reference image can be caused to almost coincide with each other. As a result, the accuracy of ultrasonic diagnosis performed by comparatively observing an ultrasonic image and a reference image captured by a medical diagnostic apparatus other than an ultrasonic diagnostic apparatus can be improved.

In addition to the first aspect, the ultrasonic diagnostic apparatus is preferably configured to comprise pressure measurement means for measuring a pressure which is applied to a body surface part of the object by the ultrasonic probe and pressure calculation means for obtaining a distribution of pressure acting on a body site in the region-of-interest on the basis of a pressure measurement value obtained by measurement by the pressure measurement means and such that the corrected ultrasonic image generation means includes enlargement ratio calculation means for obtaining a modulus of elasticity distribution of the body site in the region-of-interest on the basis of the pressure distribution in the region-of-interest calculated by the pressure calculation-means and the strain distribution in the region-of-interest and obtaining an enlargement ratio distribution for removing strain in the body site in the region-of-interest in a pressed state and performing enlargement correction on the ultrasonic image on the basis of the obtained modulus of elasticity distribution and enlargement processing means for performing enlargement correction on the ultrasonic image in the pressed state on the basis of the enlargement ratio distribution obtained by the enlargement ratio calculation means and generating the corrected ultrasonic image in the non-pressed state.

In this case, the enlargement ratio calculation means can be configured to divide the region-of-interest into a plurality of microregions in a grid pattern, obtain a modulus of elasticity of each microregion on the basis of the pressure distribution and the strain distribution in the pressed state, and obtain an enlargement ratio for removing strain in each microregion on the basis of the modulus of elasticity of the microregion, and the enlargement processing means can be configured to perform enlargement correction on each microregion in the pressed state on the basis of the enlargement ratio obtained by the enlargement ratio calculation means and generate the corrected ultrasonic image.

The strain calculation means can be configured to obtain the strain distribution only in a depth direction of the region-of-interest, and the enlargement ratio calculation means can be configured to obtain the modulus of elasticity distribution only in the depth direction of the region-of-interest and obtain the enlargement ratio distribution only in the depth direction of the region-of-interest. That is, since a compressive force applied by the ultrasonic probe has a large component in the depth direction and has a small component in a direction orthogonal to the depth direction, calculation of a correction strain distribution only in the depth direction makes it possible to shorten calculation time.

A second aspect of the present invention is an ultrasonic diagnostic apparatus characterized by comprising an ultrasonic probe which is pressed against a body surface of an object and transmits and receives an ultrasonic wave to and from the object, ultrasonic image generation means for forming an ultrasonic image on a scan plane of the ultrasonic probe on the basis of RF signal frame data of a reflected echo signal received via the ultrasonic probe, storage means for storing volume image data other than an ultrasonic image captured by an image diagnostic apparatus in advance, reference image generation means for extracting tomogram image data corresponding to the ultrasonic image from the volume image data stored in the storage means and reconstructing a reference image, and display means for displaying the ultrasonic image and the reference image on a same screen and is **characterized in that** strain calculation means for obtaining a strain distribution of a body site on the scan plane when pressed by the ultrasonic probe, on the basis of a pair of the RF signal frame data which are obtained at different measurement times and corrected reference image generation means for correcting the reference image on the basis of the strain distribution obtained by the strain calculation means and generating a corrected reference image with strain are provided, and the display means displays the ultrasonic image and the corrected reference image on the same screen.

That is, according to the second aspect of the present invention, a reference image, a corrected reference image with strain which is obtained by causing a reference image to correspond to an ultrasonic image with strain in the pressed state is generated, unlike the first aspect, and is displayed on the screen, thereby allowing accurate comparative observation.

In the second aspect of the present invention, the strain calculation means can be configured to obtain a strain distribution of a region-of-interest which is set in the ultrasonic image displayed on the screen, and the corrected reference image generation means can be configured to perform reduction processing on the reference image in the region-of-interest on the basis of the strain distribution obtained by the strain calculation means and generate the corrected reference image.

The ultrasonic diagnostic apparatus further comprises pressure measurement means for measuring a pressure which is applied to a body surface part of the object by the ultrasonic probe and pressure calculation means for obtaining a distribution of pressure acting on a body site in the region-of-interest on the basis of a pressure measurement value obtained by measurement by the pressure measurement means, and the corrected reference image generation means can be configured to include reduction ratio calculation means for obtaining a modulus of elasticity distribution of the body site in the region-of-interest on the basis of the pressure distribution in the region-of-interest calculated by the pressure calculation means and the strain distribution in the region-of-interest and obtaining a reduction ratio distribution for correcting the reference image in the region-of-interest on the basis of the obtained modulus of elasticity distribution and reduction processing means for performing reduction correction on the reference image on the basis of the reduction ratio distribution obtained by the reduction ratio calculation means and generating the corrected reference image.

In this case, the reduction ratio calculation means can be configured to divide the region-of-interest into a plurality of microregions in a grid pattern, obtain a modulus of elasticity of each microregion on the basis of the pressure distribution and the strain distribution in the pressed state, and obtain a reduction ratio for adding strain in each microregion to the reference image on the basis of the modulus of elasticity of the microregion, and the reduction processing means can be configured to perform reduction correction on a microregion of the reference image corresponding to each microregion on the basis of the reduction ratio obtained by the reduction ratio calculation means and generate the corrected reference image.

The reduction ratio calculation means can be configured to obtain the reduction ratio distribution on a pixel-by-pixel basis of the region-of-interest, and the reduction processing means can be configured to perform reduction correction on the reference image corresponding to the region-of-interest pixel by pixel on the basis of the reduction ratio distribution obtained by the reduction ratio calculation means and generate the corrected reference image. Alternatively, the reduction ratio calculation means can be configured to obtain the reduction ratio distribution on a pixel-by-pixel basis of the region-of-interest, and the reduction processing means can be configured to perform reduction correction on the reference image pixel by pixel on the basis of a redaction ratio or reduction ratios of one or adjacent ones of pixels in a depth direction of the reference image corresponding to the region-of-interest and generate the corrected reference image. In this case, the reduction processing means can be configured to combine pieces of luminance information of the adjacent ones of the pixels into a piece of luminance information for one pixel.

Brief Description of the Drawings

Figure 1 is a schematic block diagram showing an ultrasonic diagnostic apparatus according to an embodiment of the present invention;

Figures 2 are configuration views showing an embodiment of an ultrasonic probe used in the ultrasonic diagnostic apparatus according to the present invention;

Figures 3 are charts for explaining an example of operation in an enlargement processing unit according to the embodiment in Figure 1;

Figure 4 is a chart showing an example of an operation flow in the enlargement processing unit according to the embodiment in Figure 1;

Figure 5 is a view schematically showing how images obtained by the ultrasonic diagnostic apparatus according to the embodiment in Figure 1 are displayed;

Figure 6 is a schematic block diagram of an ultrasonic diagnostic apparatus according to another embodiment of the present invention;

Figures 7 are views for explaining operation of reduction processing according to the embodiment in Figure 6; and

Figures 8 are charts for explaining an example of the operation of reduction processing according to the embodiment in Figure 6.

Best Mode for Carrying Out the Invention

An ultrasonic diagnostic apparatus according to the present invention will be described below on the basis of embodiments.

First Embodiment

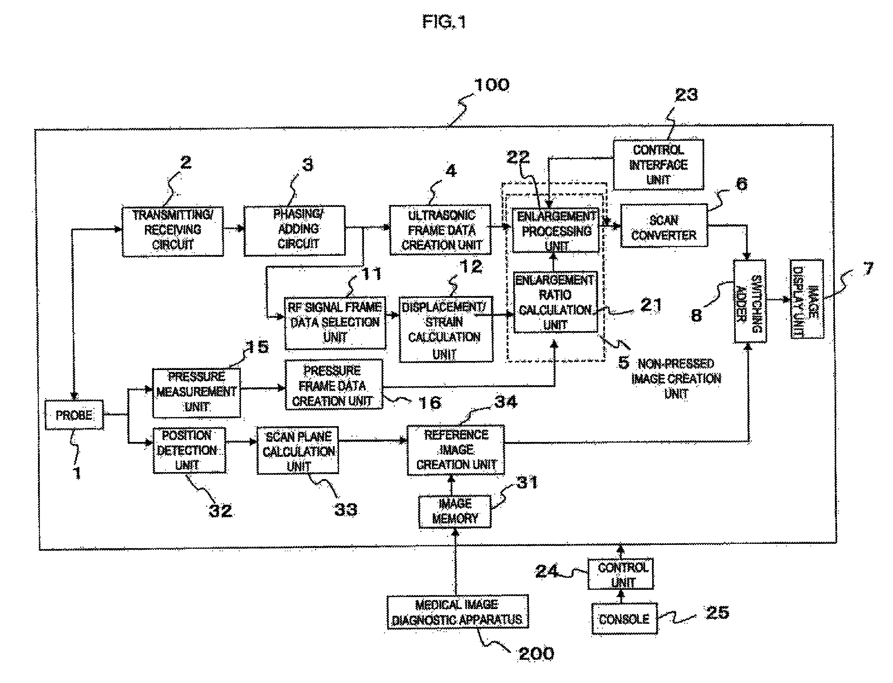

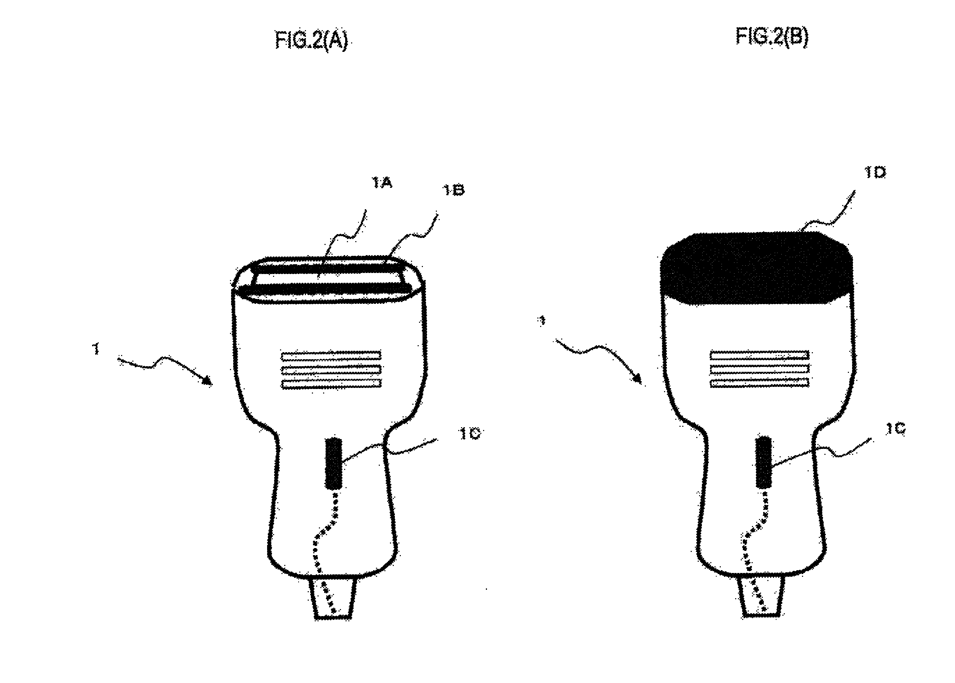

Figure 1 is a schematic block diagram of an ultrasonic diagnostic apparatus according to an embodiment of the present invention. An ultrasonic diagnostic apparatus 100 shown in Figure 1 includes an ultrasonic probe 1 which is pressed against an object (not shown) and transmits and receives an ultrasonic wave to and from the object. As shown in Figure 2(A), the ultrasonic probe 1 is configured to include a plurality of ultrasonic transducers 1A arrayed on an ultrasonic transmission/reception surface. Upon driving of a transmitting/receiving circuit 2 (to be described later), each of the ultrasonic transducers 1A are sequentially scanned. The ultrasonic transducers 1A irradiate a scan plane in an object with an ultrasonic beam and receive a reflected echo wave generated from the scan plane in the object.

The transmitting/receiving circuit 2 generates and outputs an ultrasonic pulse for generating an ultrasonic wave to each of the ultrasonic transducers 1A of the ultrasonic probe 1 and sets a convergence point of ultrasonic transmitting beam to an arbitrary depth. The transmitting/receiving circuit 2 also amplifies each of reflected echo signals received from the plurality of ultrasonic transducers 1A with a predetermined gain and then outputs the reflected echo signals to a phasing/adding circuit 3. The phasing/adding circuit 3 shifts the phases of the reflected echo signals, forms an ultrasonic receiving beam from one or a plurality of convergence points, and outputs an RF signal.

An RF signal outputted from the phasing/adding circuit 3 is inputted to an ultrasonic frame data creation unit 4 serving as ultrasonic image creation means and is subjected to gain correction, log compression, wave detection, edge enhancement, filtering, and the like. After that, ultrasonic frame data is created. The ultrasonic frame data outputted from the ultrasonic frame data creation unit 4 is inputted to a scan converter 6 via a non-pressed image creation unit 5 serving as a corrected ultrasonic image creation means. Alternatively, the ultrasonic frame data outputted from the ultrasonic frame data creation unit 4 bypasses the non-pressed image creation unit 5 and is directly inputted to the scan converter 6. Whether ultrasonic frame data is to be inputted to the scan converter 6 via the non-pressed image creation unit 5 or is to bypass the non-pressed image creation unit 5 and be inputted to the scan converter 6 can be selected by operation of a console 25 via a control unit 24.

The scan converter 6 converts inputted pieces of ultrasonic frame data having undergone A/D conversion into pieces of ultrasonic image data (tomogram image data) and stores the pieces of ultrasonic image data in a frame memory in ultrasonic cycles and sequentially reads out the pieces of ultrasonic image data in cycles for a television system. The read-out pieces of ultrasonic image data are outputted to an image display unit 7 via a switching adder 8 serving as image display means. In the image display unit 7, the inputted pieces of ultrasonic image data are D/A-converted, and then an ultrasonic image which is a tomogram image is displayed on a screen. In the above-described manner, an ultrasonic image (a B-mode image) on a scan plane where an ultrasonic beam is scanned by the ultrasonic probe 1 is reconstructed by the scan converter 6 and is displayed on the screen of the image display unit 7.

An RF signal outputted from the phasing/adding circuit 3 is also inputted to an RF signal frame data selection unit 11. The RF signal frame data selection unit 11 selects and stores a pair of pieces of RF signal frame data which are obtained on a scan plane at different measurement times. The interval between the times for the pair of pieces of RF signal frame data is arbitrarily set. The pair of pieces of RF signal frame data selected by the RF signal frame data selection unit 11 is inputted to a displacement/strain calculation unit 12.

The displacement/strain calculation unit 12 performs one-dimensional or two-dimensional correlation processing on the basis of an inputted pair of pieces of RF signal frame data and obtains a displacement or a motion vector at each measurement point on a scan plane. The displacement/strain calculation unit 12 spatially differentiates the displacement at each measurement point, calculates a strain at the measurement point, obtains a strain distribution on the scan plane as strain frame data, and outputs the strain frame data to the non-pressed image creation unit 5.

On the other hand, pressure sensors 1B are provided, e.g., at a surface of the ultrasonic probe 1 which abuts against an object in the ultrasonic probe 1, as shown in Figure 2(A). An output from each pressure sensor 1B is inputted to a pressure measurement unit 15. The pressure measurement unit 15 measures a pressure applied to the body surface of an object by the ultrasonic probel in conjunction with the pressure sensors 1B. The measured pressure is inputted to a pressure frame data creation unit 16, which estimates a pressure at each measurement point in the object, obtains a pressure distribution on a scan plane, and creates a piece of pressure frame data corresponding to each measurement point of an ultrasonic image. The pieces of pressure frame data created by the pressure frame data creation unit 16 are inputted to the non-pressed image creation unit 5.

The non-pressed image creation unit 5 is a feature of the present invention and is configured to include an enlargement ratio calculation unit 21 and an enlargement processing unit 22. The enlargement ratio calculation unit 21 assumes that no pressure is applied to a body site by the ultrasonic probe 1, i.e., that the body site is in a non-pressed state and calculates an enlargement ratio which is a strain correction amount for each measurement point, in order to remove strain indicated by a strain distribution inputted from the displacement/strain calculation unit 12. The enlargement ratios obtained by the enlargement ratio calculation unit 21 are inputted to the enlargement processing unit 22. The enlargement processing unit 22 increases, e.g., the number of pixels at each measurement point of ultrasonic frame data (an ultrasonic image) outputted from the ultrasonic frame data creation unit 4 by the corresponding enlargement ratio and created corrected ultrasonic frame data (a corrected ultrasonic image). The corrected ultrasonic frame data is converted into ultrasonic image data (tomogram image data) by the scan converter 6 and is outputted to the image display unit 7 via the switching adder B. The detailed configuration of the non-pressed image creation unit 5 will be described later together with the operation thereof.

A configuration which creates a reference image to be displayed on the image display unit 7 will be described. Volume image data (a multi-slice image) which is obtained by capturing images of the same object is stored in an image memory 31 from a medical image diagnostic apparatus 200 which is installed separately from the ultrasonic diagnostic apparatus 100 according to this embodiment and is composed of, e.g., X-ray CT equipment or MRI equipment.

On the other hand, a position sensor 1C is incorporated in the ultrasonic probe 1, as shown in Figure 2(A). The position sensor 1C is capable of detecting the three-dimensional position, the inclination, and the like of the ultrasonic probe 1. For this reason, when an ultrasonic image is captured, a signal corresponding to the position and inclination of the ultrasonic probe 1 is outputted from the position sensor 1C and is inputted to a scan plane calculation unit 33 via a position detection unit 32.

More specifically, the position sensor 1C is composed of, e.g., a sensor which detects a magnetic signal. A magnetic field source (not shown) is placed near a bed (not shown) on which an object lies. The position sensor 1C detects a magnetic field (reference coordinate system) formed in a three-dimensional space from the magnetic field source and detects the three-dimensional position and inclination of the ultrasonic probe 1. Note that although a position sensor system is composed of the position sensor 1C and the magnetic field source, the position sensor system is not limited to a system of a magnet type, and a known position sensor system such as a system using light can be used instead.

The scan plane calculation unit 33 calculates a position and an inclination in a reference coordinate system of a scan plane (sectional plane) corresponding to an ultrasonic image on the basis of a detection signal indicating the position and inclination of the ultrasonic probe 1 outputted from the position detection unit 32. The position and inclination on the scan plane obtained by the calculation are outputted to a reference image creation unit 34.

The reference image creation unit 34 extracts two-dimensional image data on a sectional plane corresponding to a position and an inclination on a scan plane from volume image data of the same object stored in the image memory 31, creates reference image data, and outputs the reference image data to the switching adder 8.

The switching adder 8 is operated in accordance with a command from the console 25, and an ultrasonic image, a corrected ultrasonic image, and a reference image are displayed in various combinations on the image display unit 7. More specifically, one of display modes, selecting one of the ultrasonic image, the corrected ultrasonic image, and the reference image and displaying the image over the display screen, displaying the corrected ultrasonic image and the reference image side by side on the display screen, and displaying the corrected ultrasonic image and the reference image superimposed on each other on the display screen, can be selected.

The detailed configuration of the non-pressed image creation unit 5, which is a feature of this embodiment, will be described together with the operation thereof. Since an ultrasonic image is obtained by pressing the ultrasonic probe 1 against the body surface of an object and transmitting and receiving an ultrasonic wave, an ultrasonic image in which a body site in the object such as an organ is deformed or strained by a compressive force applied by the ultrasonic probe 1 is generated. In contrast, since a reference image to be comparatively observed with an ultrasonic image is captured without a compressive force on an object, i.e., under only atmospheric pressure, the reference image has no strain. Accordingly, if an ultrasonic image and a reference image are displayed side by side or one superimposed on the other, the shape of a body site such as an organ in the ultrasonic image may not coincide with that of the body site in the reference image. These results prevent accurate comparative observation between the ultrasonic image and the reference image. For this reason, in this embodiment, the non-pressed image creation unit 5 corrects strain in an ultrasonic image captured in a pressed state and generates a corrected ultrasonic image in a non-pressed state, thereby allowing accurate comparative observation with a reference image.

First, the displacement/strain calculation unit 12 calculates a strain at each measurement point of RF signal frame data obtained by measurement in the pressed state and creates strain frame data representing a strain distribution. As for the strain frame data, strain calculation for creating a normal elasticity image used to diagnose a malignant tumor or the like can be applied without change. More specifically, a displacement and a strain at each measurement point are calculated using a pair of pieces of RF signal frame data stored in the RF signal frame data selection unit 11. For example, letting N be a currently stored piece of RF signal frame data, one piece X of RF signal frame data is selected among past pieces of RF signal frame data, (N-1), (N-2), (N-3) ,..., (N-M), by the RF signal frame data selection unit 11. in accordance with a control, instruction from the control unit 24. The selected piece X of RF signal frame data is temporarily stored in the RF signal frame data selection unit 11.

The displacement/strain calculation unit 12 takes in the pieces N and X of RF signal frame data in parallel from the RF signal frame data selection unit 11, performs one-dimensional or two-dimenaional correlation processing on the pair of pieces of RF signal frame data, N and X, and obtains a displacement or a motion vector at each measurement point (i,j). Here, i and j are natural numbers and represent two-dimensional coordinates. The displacement/strain calculation unit 12 spatially differentiates the obtained displacement at each measurement point (i.j), obtains a strain $\varepsilon(i,j)$ at

each measurement point, and calculates strain frame data which is a two-dimensional distribution of strain. The calculated strain frame data is inputted to the enlargement ratio calculation unit 21.

The enlargement ratio calculation unit 21 obtains a strain correction amount for removing strain in an ultrasonic image captured in the pressed state on the basis of strata frame data inputted from the displacement/strain calculation unit 12 and pressure frame data inputted from the pressure frame data creation unit 16. A strain correction amount according to this embodiment is set as an enlargement ratio for increasing the area of pixels (the number of pixels) at each measurement point in order to generate a corrected ultrasonic image in the non-pressed state. A command as to whether to cause the non-pressed image creation unit 5 to perform processing is inputted from the console 25 via the control unit 24.

Prior to description of the detailed configurations of the enlargement ratio calculation unit 21 and the enlargement processing unit 22 of the non-pressed image creation unit 5, the principles of the feature of this embodiment will be described. A strain calculated by the displacement/strain calculation unit 12 is a relative physical quantity correlating with the magnitude of a pressure acting on each measurement point of an object and the hardness of a living-body tissue at the measurement point. That is, strain becomes larger with an increase in pressure magnitude. Strain becomes large if a living-body tissue at each measurement point is soft while the strain becomes small if the living-body tissue is hard.

A modulus of elasticity representing the hardness of a living-body tissue is an absolute physical quantity which is intrinsic to a living-body tissue, regardless of the magnitude of a compressive force. Calculating a modulus of elasticity distribution on the basis of a strain distribution makes it possible to obtain a strain correction amount reflecting the hardness at each measurement point. For this reason, in this embodiment, a modulus of elasticity at each measurement point is obtained on the basis of a strain at the measurement point in the pressed state, and a strain at each measurement point with a compressive force of "0" applied by the ultrasonic probe, i.e., in the non-pressed state under atmospheric pressure is obtained on the basis of the obtained modulus of elasticity at each measurement point. Enlargement ratios are obtained as strain correction amounts from a strain distribution for the measurement points in the pressed state and a strain distribution for the measurement points in the non-pressed state, and an ultrasonic image in the pressed state is corrected on the basis of the distribution of the enlargement ratios. With this operation, it is possible to generate a corrected ultrasonic image corresponding to a reference image with high accuracy.

A concrete example will be given below. A Young's modulus will be described as an example of a modulus of elasticity. Assume that each measurement point $P_{i,j}$ represents pixel coordinates (i,j) of an ultrasonic image-Since a Young's Modulus $E_{i,j}$ of each pixel (i,j) is defined by following formula (1) using a pressure change $\Delta P_{i,j}$ and a strain $\varepsilon_{i,j}$ calculated by the displacement/strain calculation unit 12:

$$E_{i,j} = \Delta P_{i,j}/\varepsilon_{i,j} \qquad (1)$$

Since the Young's modulus $E_{i,j}$ is a value intrinsic to a living-body tissue which is irrelevant to pressure, a correction strain amount $\varepsilon'_{i,j}$ which is a total strain amount for correcting an ultrasonic image with the strain $\varepsilon_{i,j}$ in the pressed state, in which the ultrasonic probe 1 abuts against an object, to the ultrasonic image in the non-pressed state can be calculated back from the Young's modulus $E_{i,j}$ in formula (1) using formula (2) below.

In formula (2), $P1_{i,j}$ represents a pressure distribution created by the pressure frame data creation unit 16, and P0 represents a pressure at each measurement point (i,j) in the non-pressed state, in which the ultrasonic probe 1 is separated from an object, i.e., the atmospheric pressure. The pressure P0 has the same value at all measurement points (i,j).

$$\varepsilon'_{i,j} = (P1_{i,j} - P0)/E_{i,j} \qquad (2)$$

Assume that the pressure $P1_{i,j}$ attenuates in a depth direction of the ultrasonic probe 1, and a change in a line direction orthogonal to the depth direction is negligible.

An enlargement ratio $A_{i,j}$ of each pixel (i,j) for removing strain is an ultrasonic image when the pressure changes from P0 to P1 is defined by formula (3) below using the corrected strain amount $\varepsilon'_{i,j}$ in formula (2). As indicated by formula (3), if an ultrasonic image has no strain, the enlargement ratio $A_{i,j}$ becomes "1".

$$\begin{aligned}A_{i,j} &= (1 + \varepsilon'_{i,j})\\ &= \{1 + (P1_{i,j} - P0)/E_{i,j}\} \quad (3)\end{aligned}$$

Since the pressure is assumed to change only in the depth direction of the ultrasonic probe 1, a corrected ultrasonic image in the non-pressed image can be estimated by correcting each pixel (i,j) to enlarge the pixel in the depth direction by the enlargement ratio $A_{i,j}$.

The enlargement ratio calculation unit 21 calculates modulus of elasticity frame data by a calculation indicated by formula (1) using strain frame data outputted from the displacement/strain calculation unit 12 and pressure frame data outputted from the pressure frame data creation unit 16. The enlargement ratio calculation unit 21 finally calculates enlargement ratio frame data by calculations indicated by formulae (2) and (3).

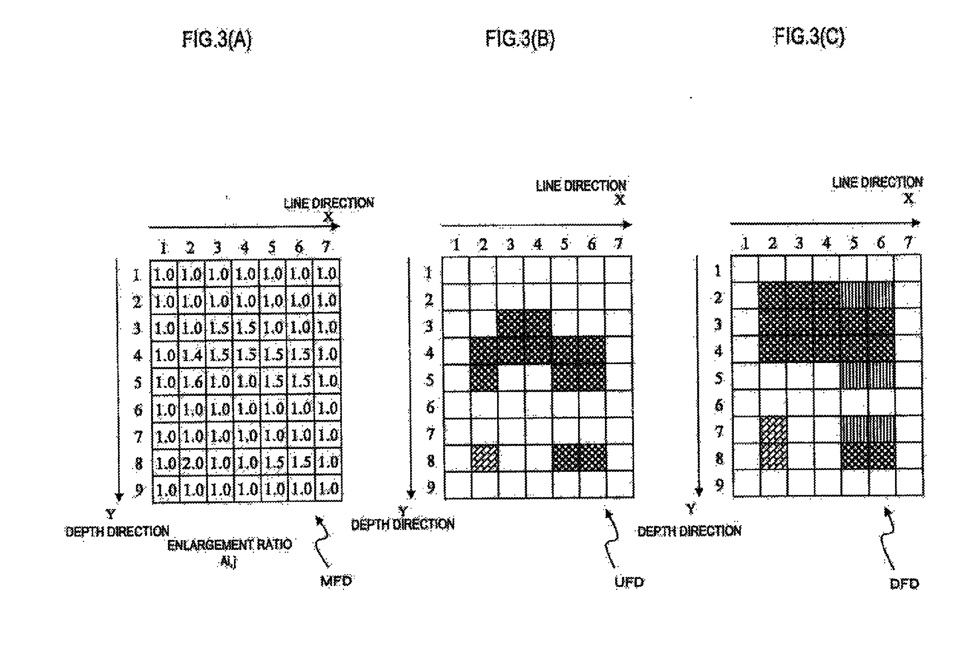

Figures 3(A) to 3(C) show for explaining an example of processing in the enlargement processing unit 22. Figure 3(A) shows enlargement ratio data MFD which is enlargement ratio data inputted from the enlargement ratio calculation unit 21 and is composed of the enlargement ratios $A_{i,j}$ stored to correspond to coordinates of ultrasonic frame data. The example shown in Figure 3(A) is a simple representation of the enlargement ratio frame data MFD. Coordinates X1 to X7 for pixels are assigned in a line direction X of a frame memory while coordinates Y1 to Y9 for pixels are assigned in a depth direction Y. For example, an enlargement ratio $A_{1,9}$ of the pixel at coordinates (1,9), 1.0, an enlargement ratio $A_{2,8}$ of the pixel at coordinates (2,8), 2.0, an enlargement ratio $A_{3,4}$ of the pixel at coordinates (3,4), 1.5, and an enlargement ratio $A_{5,8}$ of the pixel at coordinates (5,8), 1.5, are stored.

Figure 3(B) shows ultrasonic frame data inputted from the ultrasonic frame data creation unit 4. Ultrasonic frame data UFD is ultrasonic frame data on a scan plane created in the pressed state by the ultrasonic probe 1. Figure 3(C) shows corrected ultrasonic image frame data DFD which is obtained by correcting the ultrasonic frame data UFD on the basis of the enlargement ratio frame data MFD.

The procedure for creating the corrected ultrasonic image frame data DFD by the enlargement processing unit 22 is as follow. First, the enlargement ratio $A_{i,j}$ of each pair of coordinates of the enlargement ratio frame, data MFD is read out. The readout is performed sequentially, e.g., from the line coordinate X1 to the line coordinate X7 in the line direction X and from the depth coordinate Y9 with a large depth to the depth coordinate Y1 with a small depth in the depth direction Y.

In the description given with reference to Figure 3(A), readout in the depth direction Y is performed from the depth coordinate Y9. However, a depth coordinate at which readout is started can be set to an arbitrary depth coordinate Y with a smaller depth for each of line coordinates X. This is to locate a part with a strain at a part near the body surface of an object and shorten the time to create the corrected ultrasonic image frame data DFD. The read start depth coordinate can be set by, e.g., the control interface unit 23 shown in Figure 1.

As shown in Figure 3(A), at the line coordinate X1, the enlargement ratios $A_{i,j}$ for the depth coordinates Y9 to Y1 are all 1.0, and it is determined that enlargement processing need not be performed on the pixels at the depth coordinates of the line coordinate X1. Pieces of luminance information of the depth coordinates Y9 to Y1 at the line coordinate X1 of the ultrasonic frame data UFD are transferred to corresponding coordinates of the corrected ultrasonic image frame data DFD without change in destination.

At the time of readout of the enlargement ratios $A_{i,j}$ at the depth coordinates Y9 to Y1 of the line coordinate X2, since the enlargement ratio $A_{i,j}$ at the depth coordinate Y9 is 1.0, a piece of luminance information at the depth coordinate Y9 of the ultrasonic frame data UFD is transferred to a pixel at the depth coordinate Y9 of the corrected ultrasonic image frame data DFD without change in destination. Since the enlargement ratio $A_{i,j}$ at the depth coordinate Y8 is 2.0, it is determined that a corresponding pixel needs to be enlarged 2.0 times. A piece of luminance information at the depth coordinate Y8 of the ultrasonic frame data UFD is transferred to pixels at the depth coordinate Y8 and the depth coordinate Y7 of the corrected ultrasonic image frame data DFD. With these operations, the pixel at the depth coordinate Y8 of the ultrasonic frame data is enlarged 2.0 times in a body surface direction (opposite to the depth direction). Since enlargement ratios $A_{2,7}$ and $A_{2,6}$ at the depth coordinates Y7 and Y6 are 1,0, it is determined that corresponding pixels need not be Subjected to enlargement processing. In this case, since a piece of pixel information has already been written at the depth coordinate Y7 of the corrected ultrasonic image frame data DFD by the processing for the depth coordinate Y8, the transfer destination of pieces of luminance information of the pixels at the depth coordinates Y7 and Y6 is shifted, and the pieces of luminance information are transferred to pixels at the depth coordinates Y6 and Y5 of the Corrected ultrasonic image frame data DFD.

As described above, if the enlargement ratio $A_{i,j}$ is an integer, it suffices to transfer a piece of luminance information of the corresponding pixel of the ultrasonic frame data UFD to each pixel to a corresponding pixel without change in destination or shift a transfer destination to another and transfer the piece of luminance information to the pixel, in order to obtain pieces of luminance information of the corrected ultrasonic image frame data DFD. However, if the enlargement ratio $A_{i,j}$ has a fractional part, it is necessary to combine a plurality of pixels of the ultrasonic frame dada UFD and obtain pieces of luminance information of the corrected ultrasonic image frame data, DFD. Letting a1, a2 a3,... be the enlargement ratios $A_{i,j}$ of the ultrasonic frame data UFD and I1, I2, I3,.. be pieces of the luminance information of the ultrasonic frame data UFD, a formula for the combination is a formula represented by following formula (4):

$$\text{(luminance information of DFD)} = \text{(fraction part of a1)} \times I1$$
$$+ \text{(fraction part of a2)} \times I2$$
$$+ \text{(fraction part of a3)} \times I3$$
$$+ \ldots \quad (4)$$

For example, an enlargement ratio $A_{2,5}$ at the depth coordinate Y5 of the line coordinate X2 is 1.6, and an enlargement ratio $A_{2,4}$ at the depth coordinate Y4 is 1.4. It is determined that corresponding pixels need to be enlarged 1.6 times and 1.4 times, respectively. Since a piece of luminance information has already been written at the depth coordinate Y5 in the corrected ultrasonic image frame data DFD by enlargement processing, the transfer destinations of pieces of luminance information at the depth coordinates Y5 and Y4 of the ultrasonic frame data UFD are shifted, and the pieces of luminance information are transferred to pixels at the depth coordinates Y4, Y3, and Y2. At this time, the piece of luminance information at the depth coordinate Y5 of the ultrasonic frame data UFD is transferred to the pixel at the depth coordinate Y4 in the corrected ultrasonic image frame data DFD. A combined value of the pieces of luminance information at the depth coordinates Y5 and Y4 of the ultrasonic frame data UFD is transferred to the pixel at the depth coordinate Y3 in the corrected ultrasonic image frame data DFD. That is, the combination is performed using formula (4) by calculating (luminance information at Y5 of UFD) $\times$ (0.6) + (luminance information at Y4 of UFD) $\times$ (0.4). Finally, the piece of luminance information at the depth coordinate Y4 of the ultrasonic frame data UFD is transferred to the pixel at the depth coordinate Y2 in the corrected ultrasonic image frame data DFD.

As for the line coordinate X5, the enlargement ratio $A_{5,8}$ at the depth coordinate Y8 of the line coordinate X5 is 1.5, and the enlargement ratio $A_{5,7}$ at the depth coordinate Y7 is 1.0. Although corresponding pixels need to be enlarged 1.5 times and 1.0 times, respectively, the number of pixels can only be an integer.

For this reason, the enlargement processing unit 22 first transfers a luminance value at the depth coordinate Y8 of the ultrasonic frame data UFD is transferred to a pixel at the depth coordinate Y8 in the corrected ultrasonic image frame data DFD.

A combined value of pieces of luminance information at the depth coordinates Y7 and Y8 of the ultrasonic frame data UFD is transferred to a pixel at the depth coordinate Y7. More specifically, since the pixel at the depth coordinate Y8 is enlarged 1.5 times, an enlargement corresponding to 0.5 times the pixel is pushed out to the depth coordinate Y7. For this reason, as for the pixel at the depth coordinate Y7, the combination is performed by calculating (luminance information at Y7 of UFD) $\times$ (0.5) + (luminance information at Y8 of UFD) $\times$ (0.5).

An enlargement ratio $A_{5,6}$ at the depth coordinate Y6 is 1.0. A combined value of pieces of luminance information at the depth coordinates Y6 and Y7 of the ultrasonic frame data UFD is transferred to a pixel at the depth coordinate Y6. More specifically, an enlargement corresponding to 0.5 times the pixel at the depth coordinate Y7 is pushed out to the depth coordinate Y6. For this reason, as for the pixel at the depth coordinate Y6, the combination is performed by calculating (luminance information at Y6 of UFD) $\times$ (0.5) + (luminance information at Y7 of UFD) $\times$ (0.5).

An enlargement ratio $A_{5,5}$ at the depth coordinate Y5 is 1.5. A combined value of pieces of luminance information at the depth coordinates Y5 and Y6 of the ultrasonic frame data UFD is transferred to a pixel at the depth coordinate Y5. More specifically, the combination is performed by calculating (luminance information at Y5 of UFD) $\times$ (0.5) + (luminance information at Y6 of UFD) $\times$ (0.5). A value 1.0 times a luminance value at the depth coordinate Y5 of the ultrasonic frame data UFD is transferred to a pixel at the depth coordinate Y4 in the corrected ultrasonic image frame data DFD.

As described above, by repeating the above-described processing until the line coordinate X7, the corrected ultrasonic image frame data DFD shown in Figure 3(C) is created. The corrected ultrasonic image frame data DFD is outputted to the scan converter 6 shown in Figure 1 frame by frame, and a corrected ultrasonic image in the non-pressed state is displayed on the screen of the image display unit 7.

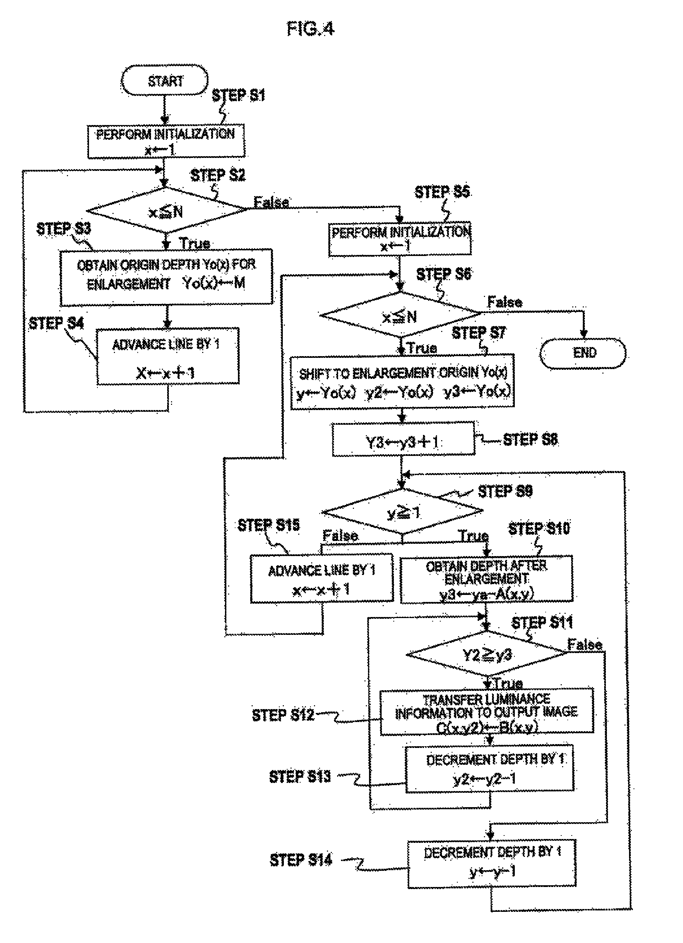

Figure 4 shows a flow chart as an example of the processing operation of the above-described enlargement processing unit 22. In step S1 of Figure 4, a line coordinate X of a frame memory is initialized to 1. In step S2, it is determined whether the line coordinate X is not more than a maximum value N for the number of lines. It the line coordinate X is not more than the maximum value N, the flow advances to step S3 to determine an origin depth $Y_0(X)$ for enlargement processing. The origin depth $Y_0(X)$ is set by the control interface unit 23 shown in Figure 1 and is the depth coordinate Y9 in the example of Figures 3. In step S4, the line coordinate X is incremented by 1 and advances by 1. Steps S2, S3, and S4 are repeated until the line coordinate X becomes larger than the maximum value N. That is, the origin depth $Y_0(X)$ for enlargement processing on the frame memory is set for each value of the line coordinate X by the processes in steps S2 to S4.

When the process of determining the origin depth $Y_0(X)$ for each value of the line coordinate X ends, the flow advances to step S5 to initialize the line coordinate X of the frame memory to 1. It is determined in step S6 whether the line coordinate X is not more than the maximum value N. If the line coordinate X is not more than the maximum value N, the flow advances to step S7 to initialize a coordinate y of the ultrasonic frame data UFD, a coordinate y2 of the corrected ultrasonic image frame data DFD, and a primary variable y3 used to calculate y2 to the origin depth $Y_0(X)$. In step S8, y3 is incremented by 1. In step S9, it is determined whether y is not less than 1. If it is determined that y is not less than 1, the post-enlargement depth y3 is calculated by (y3 - A(x,y)) in step S10. In the formula, A(x,y) represents an enlargement ratio at coordinates (x,y) of the enlargement ratio frame data and is identical to $A_{i,j}$ described above. In step S11, it is determined whether y2 is not less than y3.

If it is determined in the determination in step S11 that y2 is not less than y3, a piece of luminance information of a pixel B(x,y) in the ultrasonic frame data UFD is transferred to a corresponding pixel C(x,y2) of the corrected ultrasonic image frame data DFD, which is an output image in step S12. In step S13, the depth coordinate y of the ultrasonic frame data UFD is decremented by 1, and the flow returns to step S11. In step S11, it is determined whether y2 is not less than y3, as described above. If y2 is less than y3, the flow advances to step S14. In step S14, the depth coordinate y of the corrected ultrasonic image frame data DFD is decremented by 1, and the flow returns to step S9. In this manner, if it is determined in step S9 that y is not less than 1, the processes in steps S10, S11, S12, S13, and S14 are repeated until y becomes less than 1.

If it is determined in the determination in step S9 that y is less than 1, the flow advances to step S15. In step S15, X is incremented by 1, and the line coordinate X advances by 1. The flow returns to step S6 to repeat the above-described processes. That is, it is determined in step S6 whether X is not more than the maximum value N. The above-described operation is repeated if X is not more than the maximum value N, and the process ends if X exceeds the maximum value N.

As described above, by performing enlargement processing by the procedure shown in Figure 4, it is possible to create the corrected ultrasonic image frame data shown in Figure 3(C).

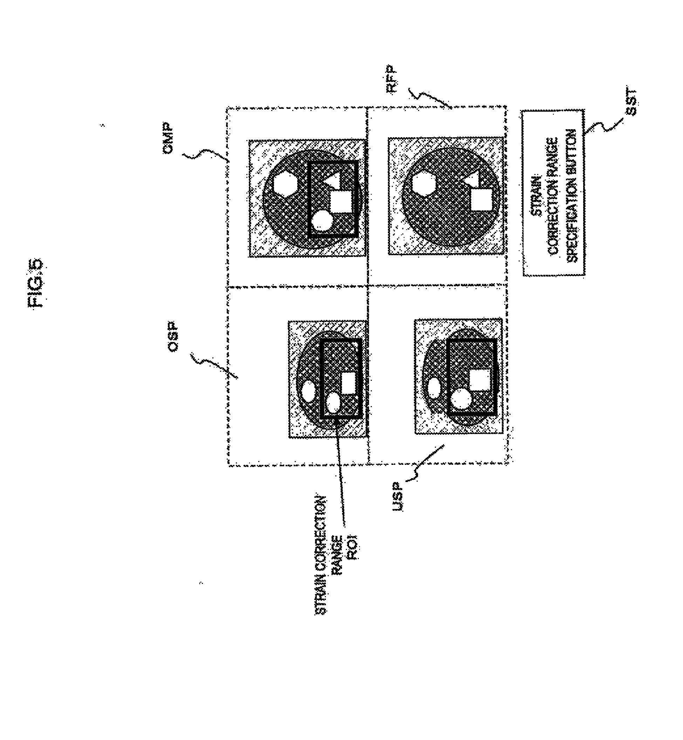

Figure 5 shows an example of an image displayed on the image display unit 7 by the ultrasonic diagnostic apparatus according to this embodiment. As shown in Figure 5, an ultrasonic image OSP captured in the pressed state is displayed in an upper left display region of the screen of the image display unit 7, a corrected ultrasonic image USP in the non-pressed state which has undergone correction is displayed in a lower left display region, a reference image RFP is displayed in a lower right display region, and a composite image CMP which is obtained by superimposing the corrected ultrasonic image USP and the reference image RFP on each other is displayed side by side in an upper right display region.

As described above, according to this embodiment, it is possible to accurately observe the corresponding positions of, e.g., an organ of the corrected ultrasonic image USP and the reference image RFP and the relationship between the shapes of the organ by observing the composite image CMP shown in Figure 5.

The screen of the image display unit 7 shown in Figure 5 according to this embodiment is provided with the function of setting the enlargement origin depth $Y_0(X)$ shown in step S3 of Figure 4. That is, an operator can set the line coordinate X at the enlargement origin depth $Y_0(X)$ on the ultrasonic image OSP by a mouse operation. The screen is configured to allow setting of a strain correction range across which strain removal is performed as a region-of-interest, ROI. By clicking a specification button SST displayed on the screen, the ROI is fixed. Setting the ROI serving as the strain correction range as a region (a region on the memory) to be corrected shown in Figure 3(A) makes it possible to locate a part where strain locally occurs and shorten arithmetic processing time in the enlargement ratio calculation unit 21 and the enlargement processing unit 22.

Note that, as for setting of the ROI serving as the strain correction range, for example, the boundary of the ROI is drawn by a pointing device or the like on the ultrasonic image OSP, information on the boundary is associated with coordinates of the ultrasonic image frame data, and the coordinates are inputted from the control interface unit 23 shown in Figure 1 to the non-pressed image creation unit 5.

As has been described above, according to this embodiment, the displacement/strain calculation unit 12 obtains a strain distribution of a body site on a scan plane in the pressed state, in which a pressure is applied by the ultrasonic probe 1, and the non-pressed image creation unit 5 corrects an ultrasonic image and generates a corrected ultrasonic image in the non-pressed state, in which no pressure is applied to the body site, such that strain is removed on the basis of the obtained strain distribution. Accordingly, accuracy when measuring, e.g., the distance to, the area of, and the volume of each site of a living body on the basis of an ultrasonic image can be improved.

A corrected ultrasonic image in the non-pressed state can be displayed on the same screen as a reference image. It is thus possible to cause the shape of a body site such as an organ in a corrected ultrasonic image to coincide with that of the body site in a reference image and improve the accuracy of ultrasonic diagnosis performed by comparatively observing an ultrasonic image and a reference image captured by a medical diagnostic apparatus other than an ultrasonic diagnostic apparatus.

The pressure measurement unit 15 and the pressure frame data creation unit 16, which obtains the distribution of pressure acting on a body site as an ROI on the basis of a pressure measurement value obtained by measurement by the pressure measurement unit 15, are further provided. In the non-pressed image creation unit 5, a modulus of elasticity distribution of a body site as an ROI is obtained on the basis of a pressure distribution and a strain distribution of the ROI, strain in the body site as the ROI in the pressed state is removed on the basis of the obtained modulus of elasticity distribution, an enlargement ratio distribution for enlargement and correction of an ultrasonic image is obtained, and the ultrasonic image in the pressed state is enlarged and corrected on the basis of the obtained enlargement ratio distribution. Accordingly, a corrected ultrasonic image from which strain in an ultrasonic image has been in the pressed state removed with high accuracy can be obtained.

A compressive force applied by the ultrasonic probe 1 has a large component in the depth direction and has a small component in a direction orthogonal to the depth direction. In consideration of this, the displacement/strain calculation unit 12 and the enlargement ratio calculation unit 21 obtain a strain distribution and a modulus of elasticity distribution only in the depth direction of an ROI and obtain an enlargement ratio distribution only in the depth direction of the ROI. Accordingly, calculation time can be shortened.

Although a corrected ultrasonic image is created by performing enlargement in units of pixels in the above-described first embodiment, the present invention is not limited to this. It is also possible to set a microregion composed of a plurality of pixels, perform enlargement in units of microregions, and create a corrected ultrasonic image. That is, the enlargement ratio calculation unit 21 divides a region-of-interest into a plurality of microregions in a grid pattern, obtains the modulus of elasticity of each microregion on the basis of a pressure distribution and a strain distribution in the pressed state, and obtains an enlargement ratio for removing strain in each microregion on the basis of the modulus of elasticity of the microregion. The enlargement processing unit 22 is configured to enlarge and correct each microregion in the pressed state on the basis of the enlargement ratio and generate a corrected ultrasonic image.

In the above-described first embodiment, an example has been described in which the pressure sensors 1B are provided at the ultrasonic probe 1 to detect a pressure applied by the ultrasonic probe 1, as shown in Figure 2(A). The present invention is not limited to this, and a configuration in which a reference deformable body 1D whose modulus of elasticity is known is provided on the ultrasonic transmission/reception surface of the ultrasonic transducers 1A can be adopted, as shown in, e.g., Figure 2(B). With this configuration, when an image is captured by pressing the ultrasonic transducers 1A against the body surface of an object, an ultrasonic image of the reference deformable body 1D is obtained. Accordingly, measurement of a strain in the reference deformable body 1D makes it possible to calculate a pressure applied by the ultrasonic probe 1 using following formula (5):

$$(\text{pressure}) = (\text{strain in reference deformable body}) / (\text{ modulus of elasticity of reference deformable body}) \qquad (5)$$

Note that attenuation of pressure in the depth direction of an object can be estimated using data such as an empirical value.

Second Embodiment

In the first embodiment, a corrected ultrasonic image which is obtained by correcting an ultrasonic image to have no strain and a reference image are comparatively observed. The present invention, however, is not limited to this. As in a second embodiment to be described below, the same advantages can be achieved even if a reference image and an ultrasonic image are comparatively observed after adding, to a reference image, a strain equivalent to one in an ultrasonic image.

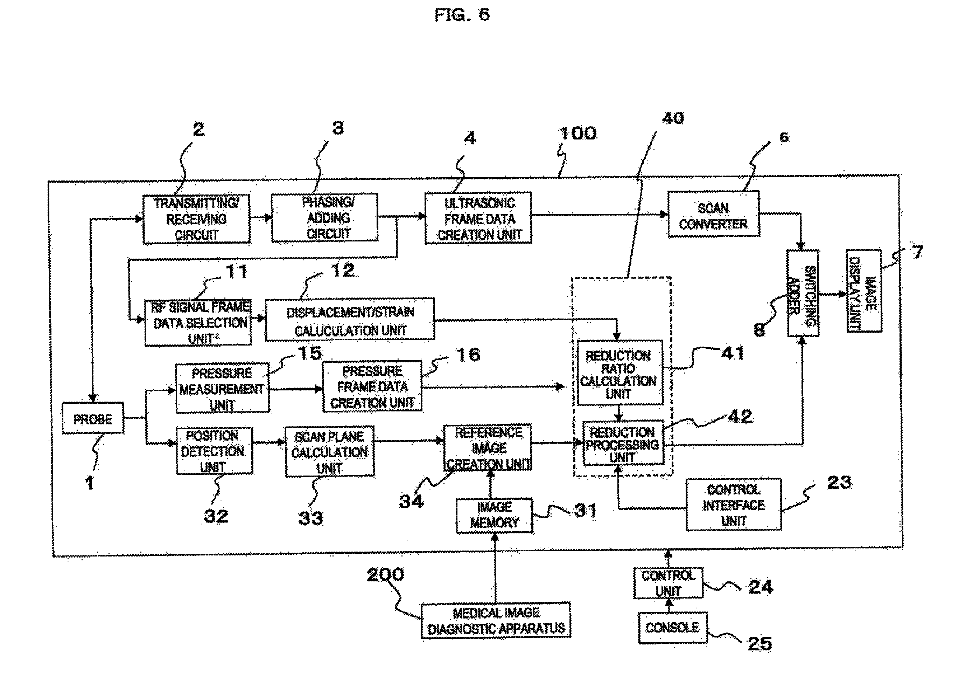

Figure 6 shows a block diagram of the second embodiment of an ultrasonic diagnostic apparatus according to the present invention. In Figure 6, a block having the same functional configuration as in Figure 1 is denoted by the same reference numeral, and a description thereof will be omitted. Figure 6 is different from Figure 1 in that ultrasonic frame data outputted from an ultrasonic frame data creation unit 4 is inputted to an image display unit 7 via a scan converter 6 and a switching adder 8. With this configuration, an ultrasonic image with strain added by an ultrasonic probe 1 is displayed on the image display unit 7 without change.

A pressed image creation unit 40 for correcting a reference image to an ultrasonic image in a pressed state is configured to include a reduction ratio calculation unit 41 and a reduction processing unit 42. To the reduction ratio calculation unit 41, strain frame data is inputted from a displacement/strain calculation unit 12, and pressure frame data is inputted from a pressure frame data creation unit 16. A reference image created by a reference image creation unit 34 is inputted to the reduction processing unit 42. The reduction processing unit 42 reduces the reference image on the basis of reduction ratio distribution data inputted from the reduction ratio calculation unit 41 and outputs a reference image with a strain equivalent to one in an ultrasonic image in a pressed state to the image display unit 7 via the switching adder 8.

The detailed configuration of the reduction ratio calculation unit 41 will be described together with the operation thereof. Assume, in this embodiment as well, that a displacement and a strain in a living-body tissue due to pressure applied by the ultrasonic probe 1 occur only in a depth direction, and a displacement and a strain in a line direction orthogonal to the depth direction are negligible. The process of thinning out pixels of a reference image in the depth direction and reducing, e.g., the number of pixels with the same luminance in the depth direction is required to strain the reference image to correspond to an ultrasonic image. For this reason, reduction processing according to this embodiment is performed in units of microregions $S_{i,j}$, each composed of a plurality of pixels in the depth direction. Each microregion $S_{i,j}$ has one pixel in a line direction and a plurality of (n) pixels in the depth direction, the number (n) of which is inputted and set in advance from a console 25.

Accordingly the reduction ratio calculation unit 41 obtains an average strain $\varepsilon_{S(i,j)}$ for each of the set microregions $S_{i,j}$ on the basis of strain frame data inputted from the displacement/strain calculation unit 12. The reduction ratio calculation unit 41 also obtains an average modulus of elasticity $E_{S(i,j)}$ for each of the microregions $S_{i,j}$ on the basis of pressure frame data inputted from the pressure frame data creation unit 16. The reduction ratio calculation unit 41 obtains a correction strain amount $\varepsilon'_{i,j}$ by formula (2) above and obtains a reduction ratio $R_{i,j}$ for a reference image in the depth direction by following formula (6):

$$R_{i,j} = (1 - \varepsilon'_{i,j})$$
$$= \{1 - (P1_{i,j} - P0)/E_{0(i,j)}\} \qquad (6)$$

The reduction processing unit 42 reduces the number of pixels in each microregion $S_{i,j}$ of a reference image inputted from the reference image creation unit 34 according to the reduction ratio $R_{i,j}$ calculated by the reduction ratio calculation unit 41, thereby adding strain to the reference image to correspond to strain in an ultrasonic image in the pressed state and creating a corrected reference image.

The created corrected reference image is outputted to the image display unit 7 via the switching adder 8, In the same manner as in Figure 5, at least an ultrasonic image and a corrected reference image are displayed side by side or are displayed while being superimposed on each other.

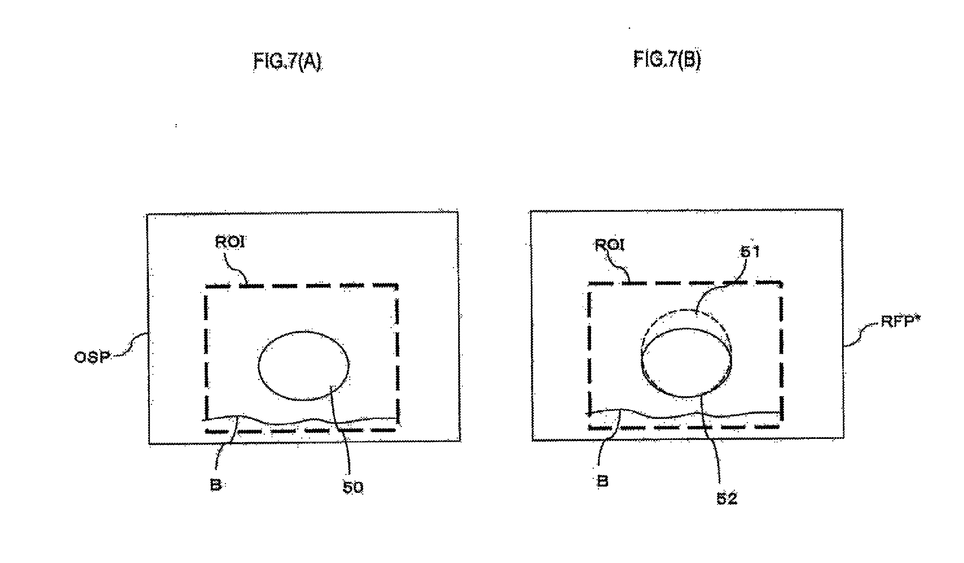

Coordinate alignment of an ultrasonic image and a reference image in the reduction processing unit 42 will be described. As has been described in the first embodiment a reference image is created by acquiring a tomogram image on the same scan plane as an ultrasonic image in the reference image creation unit 34. At this time, coordinate alignment of the ultrasonic image and the reference image in a three-dimensional spatial coordinate system is performed with respect to an object. As a result, an ultrasonic image USP and a reference image RFP displayed on the image display unit 7 are displayed at almost the same position of the screen, as shown in Figures 7(A) and 7(B), respectively. An ROI as a strain correction range which is set on the ultrasonic image USP can also be set at almost the same position on the reference image RFP.

However, it is desirable to set, as a reference, a line or a region common to an ultrasonic image and a reference image in order to improve the correction accuracy for a corrected reference image in the reduction processing unit 42. The value of a pressure applied by the ultrasonic probe 1 attenuates and becomes negligible with an increase in a depth in an object. For this reason, the correction accuracy can be improved by setting a reference line B at a position with a large depth in an ROI on the image at the boundary between different observable living-body tissues, as shown in Figure 7(A).

The setting of the reference line B is performed as in the case of ROI setting. An operator displays the ultrasonic image USP on the image display unit 7 and inputs a command through a control interface unit 23, thereby performing the setting, Note that the reference line B has the same technical meaning as the origin depth $Y_0(X)$ in the first embodiment.

The reduction processing unit 42 uses the set reference line B as a base point, reduces the number of pixels in each microregion $S_{i,j}$ according to the reduction ratio $R_{i,j}$ calculated by the reduction ratio calculation unit 41, and creates a corrected reference image. The creation of a corrected reference image is performed by storing reduction ratio frame data, ultrasonic frame data UFD, and corrected reference frame data in a frame memory, as described with reference to Figures 3(A) to 3(C). The number of pixels is a natural number. If the reduction ratio $R_{i,j}$ has a fractional part, it may be impossible to reduce the number of pixels in one microregion $S_{i,j}$ according to the reduction ratio $R_{i,j}$. In this case, coordination between the microregion $S_{i,j}$ and each of the microregion $S_{i,j-1}$ and the microregion $S_{i,j+1}$ adjacent in the depth direction is performed.

By creating a corrected reference image as described above, strain is added to a body site 51 of a reference image corresponding to a body site 50 of an ultrasonic image OSP, and a corrected reference image RFP* having a body site 52 equal in shape to the body site 50 of the ultrasonic image OSP is created, as shown in Figures 7(A) and 7 (B), It is thus possible to accurately perform comparative observation of an ultrasonic image and a corrected reference image.

Third Embodiment

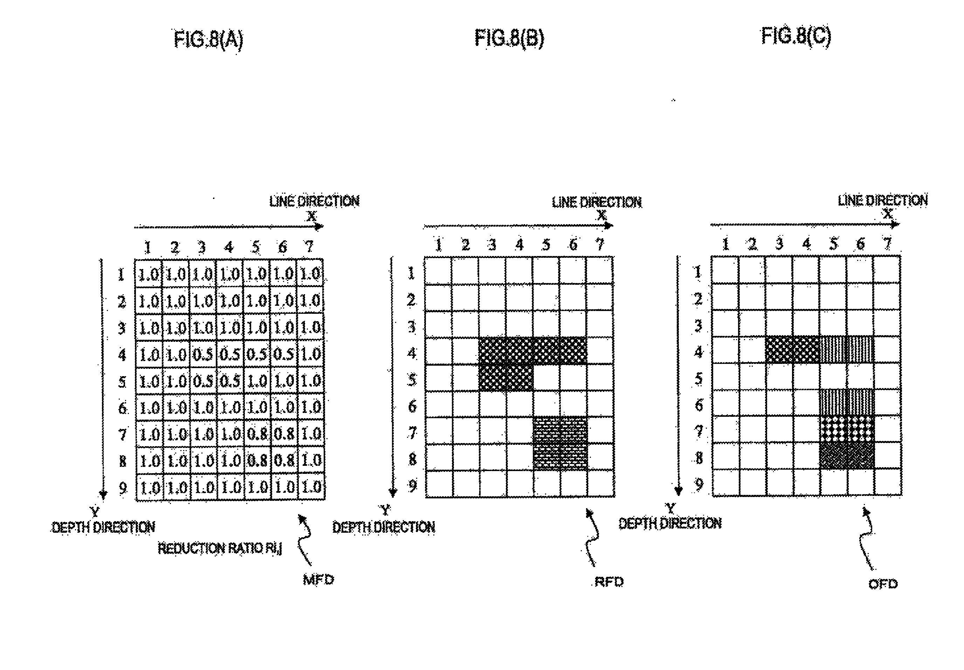

Although a reference image is corrected on the basis of a microregion in the second embodiment, a reference image can be corrected line by line.
More specifically, at line coordinates X1 and X2, redaction ratios $R_{i,j}$ at depth coordinates Y1 to Y9 are all 1.0, as shown in Figure 8(A). Accordingly, it is determined that reduction processing need not be performed on pixels at the depth coordinates of the line coordinates X1 and X2. Pieces of luminance information at the depth coordinates Y1 to Y9 of the line coordinates X1 and X2 of reference image frame data RFD are transferred to corresponding coordinates of corrected reference image frame data OFD without change. That is, although enlargement processing is performed from the depth coordinate Y9 with a large depth to the depth coordinate Y1 with a small depth in the first embodiment, redaction processing is performed from the depth coordinate Y1 with the small depth to the depth coordinate Y9 with the large depth.
At a line coordinate X3, the reduction, ratios $R_{i,j}$ at the depth coordinates Y1 to Y3 are all 1.0. Accordingly, pieces of luminance information at the depth coordinates Y1 to Y3 of the reference image frame data RFD are transferred two pixels at the depth coordinates Y1 to Y3 of the corrected reference image frame data OFD without change. Since the reduction ratios $R_{i,j}$ at the depth coordinates Y4 and Y5 are 0.5, corresponding pixels need to be reduced 0.5 times. Pieces of luminance information at the depth coordinates Y4 and Y5 of the reference image frame data RFD are thus transferred to a pixel at the depth coordinate Y4 of the corrected reference image frame data OFD. More specifically, as for the pixel at the depth coordinate Y4, the combination is performed by calculating (luminance information at Y4 of OFD) x (0.5) + (luminance information at Y5 of OFD) x (0.5).
Since a reduction ratio $R_{3,6}$ at the depth coordinate Y6 is 1.0, reduction processing needs not be performed on a pixel art the depth coordinate Y6, and a piece of luminance information is transferred to a pixel at the depth coordinate Y5 which is not filled due to the reduction. In the same manner, reduction processing is not performed for each of the depth coordinates Y7 to Y9, and pixels are transferred.
As described above, if the reduction ratio $R_{i,j}$ has a fractional part (is not more than 1.0), it is necessary to combine a plurality of pixels of the reference image frame data RFD and use the result as a piece (or pieces) of luminance information of the corrected reference image frame data OFD.
Since, at the line coordinate X5, the reduction ratios $R_{i,j}$ at the depth coordinates Y1 to Y3 are 1.0, pieces of luminance information at the depth coordinates Y1 to Y3 of the reference image frame data RFD are transferred to pixels at the depth coordinates Y1 two Y3 of the corrected reference image frame data OFD without change.
A reduction ratio $R_{5,4}$ at the depth coordinate Y4 of the line coordinate X5 is 0.5, and a reduction ratio $R_{5,5}$ at the depth coordinate Y5 is 1.0. In the reduction processing unit 42, a combined value of pieces of luminance information at the depth coordinates Y4 and Y5 of the reference image frame data RFD is transferred to a pixel at the depth coordinate Y4, More specifically, since a pixel at the depth coordinate Y4 is reduced 0.5 times, a piece of pixel information at the depth coordinate Y4 is short by 0.5. times the original pixel. For this reason, the combination is performed for the pixel at the depth coordinate Y4 by calculating (luminance information at Y4 of OFD) $\times$ (0.5) + (luminance information at Y5 of OFD) $\times$ (0.5).
The reduction ratio $R_{5,5}$ at the depth coordinate Y5 is 1.0. A combined value of pieces of luminance information at the depth coordinates Y5 and Y6 of the reference image frame data RFD is transferred to a pixel at the depth coordinate Y5. More specifically, since 0.5 times the pixel at the depth coordinate Y5 is pushed out to the depth coordinate Y4, the combination is performed for the pixel at the depth coordinate Y5 by calculating (luminance information at Y5 of OFD) $\times$ (0.5) + (luminance information at Y6 of OFD) $\times$ (0.5).
A redaction ratio $R_{5,5}$ at the depth coordinate Y6 is 1.0. A combined value of pieces of luminance information at the depth coordinates Y6 and Y7 of the reference image frame data RFD is transferred to a pixel at the depth coordinate Y6. More specifically, since 0.5 times the pixel at the depth coordinate Y6 is pushed out to the depth coordinate Y5, the combination is performed by calculating (luminance information at Y6 of OFD) $\times$ (0.5) + (luminance information at Y7 of OFD) $\times$ (0.5).

A reduction ratio $R_{5,7}$ at the depth coordinate Y7 is 0.8. A combined value of pieces of luminance information at the depth coordinates Y7 and Y8 of the reference image frame data RFD is transferred to a pixel at the depth coordinate Y7. More specifically, since 0.5 times the pixel at the depth coordinate Y7 is pushed out to the depth coordinate Y6, the combination is performed by calculating (luminance information at Y7 of OFD) $\times$ (0.3) + (luminance information at Y8 of OFD) $\times$ (0.7).

A reduction ratio $R_{5,7}$ at the depth coordinate Y8 is 1.0. A combined value of pieces of luminance information at the depth coordinates Y8 and Y9 of the reference image frame data RFD is transferred to a pixel at the depth coordinate Y8. More specifically, since 0.7 times the pixel at the depth coordinate Y8 is pushed out to the depth coordinate Y7, the combination is performed by calculating (luminance information art Y7 of OFD) $\times$ (0,1) + (luminance information at Y8 of OFD) $\times$ (0.9).

By repeating the above-described processes until a line coordinate X7, the corrected reference image frame data OFD is cremated, as shown in Figure 8(C). The corrected reference image frame data OFD is outputted frame by frame, and a corrected reference image is displayed on a screen of an image display unit 7.

That is, according to this embodiment, a reduction ratio calculation unit 41 obtains a reduction ratio distribution on a pixel-by-pixel basis of a region-of-interest, ROT, A reduction processing unit 42 performs reduction correction on a reference image in units of pixels on the basis of the reduction ratio or ratios of one pixel or a plurality of adjacent pixels in the depth direction of the reference image corresponding to the region-of-interest, ROI, and generates a corrected reference image. In this case, the reduction processing unit 42 can combine pieces of luminance information of the plurality of adjacent pixels and reduce the result to one pixel.

By creating a corrected reference image as described above, strain is added to a body site 51 of a reference image corresponding to a body site 50 of an ultrasonic image OSP, a corrected reference image RFP* having a body site 52 equal in shape to the body site 50 of the ultrasonic image OSP is created, as in the example shown in Figures 7(A) and 7(B). It is thus possible to accurately perform comparative observation of an ultrasonic image and a corrected reference image.

Fourth Embodiment

The first embodiment has illustrated an example in which the enlargement ratio $A_{i,j}$ at each pixel (i,j) is obtained by formula (3) to correct an ultrasonic image with a strain $\varepsilon 1_{i,j}$ in a pressed state under the pressure $P1_{i,j}$ to an ultrasonic image in the non-pressed state under the pressure P0 using the modulus of elasticity $E_{i,j}$ at each measurement point, and a corrected ultrasonic image in a non-pressed state is created in accordance with the procedures shown in Figures 3(A) to 3(C).

The second and third embodiments have illustrated examples in which the reduction ratio $R_{i,j}$ at each pixel (i, j) is obtained by formula (6) to add a strain to one in an ultrasonic image in the pressed state to a reference image, and a corrected reference image in the pressed state is created.

A fourth embodiment of the present, invention is **characterized in that** a corrected ultrasonic image or a corrected reference image is created without using a modulus of elasticity $E_{i,j}$ thereby shortening arithmetic processing time. Strain in a living-body tissue caused by a compressive force applied by an ultrasonic probe 1 is related to a pressure applied to the living-body tissue and the modulus of elasticity of the living-body tissue, and the modulus of elasticity of a body tissue is an absolute value which is intrinsic to the tissue. Strain in a living-body tissue depends on a pressure applied to the living-body tissue. Accordingly, if a compressive force applied by the ultrasonic probe 1 remains constant or falls within a certain range, a correction strain amount $\varepsilon'_{i,j}$ remains constant or falls within a certain range. For this reason, the enlargement ratio calculation unit 21 according to the first embodiment may obtain the enlargement ratios $A_{i,j}$ by formula (7) below on the basis of a distribution of strain s $\varepsilon_{i,j}$ at measurement paints outputted from the displacement/strain calculation unit 12. In formula (7), $\alpha$ is a correction coefficient which is set according to a pressed condition in order to convert the strain $\varepsilon_{i,j}$ into the correction strain amount $\varepsilon'_{i,j}$. Note that the correction coefficient $\alpha$ can be variably set according to how a corrected ultrasonic image and a reference image are shifted from each other when the two images are comparatively displayed or displayed while being superimposed on each other.

$$A_{i,j} = (1 + \alpha \cdot \varepsilon_{i,j}) \qquad\qquad (7)$$

On the basis of the enlargement ratio obtained in the above-described manner, the number of pixels of each measurement point is increased according to the enlargement ratio $A_{i,j}$ with respect to a strain at an origin depth Y(0), as in the first embodiment. This makes it possible to create a corrected ultrasonic image similar to one in the first embodiment.

The reduction ratio calculation unit 41 according to the second or third embodiment may obtain the reduction ratios $R_{i,j}$ by formula (8) below on the basis of a distribution of the strains $\varepsilon_{i,j}$ at the measurement points outputted from the displacement/strain calculation unit 12. In formula (8), $\beta$ is a correction coefficient which is set according to the pressed condition in order to convert the strain $\varepsilon_{i,j}$ into the correction strain amount $\varepsilon'_{i,j}$. Note that the correction coefficient $\beta$ can be variably set recording to how an ultrasonic image and a corrected reference image are shifted from each other when the two images are comparatively displayed or displayed while being superimposed on each other.

$$R_{i,j} = (1 - \beta \cdot \varepsilon_{i,j}) \tag{8}$$

Additionally, it is preferable to variably set the correction coefficients $\alpha$ and $\beta$ on the basis of a pressure distribution outputted from a pressure frame data creation unit 16.

As described above, according to this embodiment, if a pressure $P1_{i,j}$ in a pressed state falls within a certain range, a corrected ultrasonic image or a corrected reference image from which strain has been removed with certain accuracy can be obtained.

Since calculation of a modulus of elasticity and/or calculation of a pressure distribution can be omitted, the time for correction processing on an ultrasonic image or a reference image can be shortened.

Note that although the above-described first to fourth embodiments have been described in the context of a B-mode image as an ultrasonic image, an ultrasonic image according to the present invention is not limited to a B-mode image. Any other image such as a CFM image or an elasticity image may be used.

An elasticity image formation unit which forms color elasticity image data on the basis of a strain distribution calculated by a displacement/strain calculation unit 12 or elasticity information distribution calculated by an enlargement ratio calculation unit 71 can be provided. A color elasticity image can be displayed on a screen of an image display unit 7 by providing a color scan converter and converting color elasticity image data outputted from the elasticity image formation unit into a color elasticity image. It is possible to display an ultrasonic image and a color elasticity image superimposed on each other or display the images side by side by a switching adder 8.

In the case of the first embodiment, it is also possible to perform enlargement processing on a color elasticity image by an enlargement processing unit 22 and display an enlarged color elasticity image on the screen of the image display unit 7.

## Claims

1.    An ultrasonic diagnostic apparatus **characterized by** comprising an ultrasonic probe which is pressed against a body surface of an object and transmits and receives an ultrasonic wave to and from the object, ultrasonic image generation means for forming an ultrasonic image on a scan plane of the ultrasonic probe on the basis of RF signed frame data of a reflected echo signal received via the ultrasonic probe, and display means for displaying the ultrasonic image on a screen,
     wherein strain calculation means for obtaining a strain distribution of a body site on the scan plane when pressed by the ultrasonic probe, on the basis of a pair of the RF signal frame data which are obtained at different measurement times and
     corrected ultrasonic images generation means for generating a corrected ultrasonic image in a non-pressed state in which no pressure is applied to the body site, on the basis of the strain distribution obtained by the strain calculation means are provided, and
     the display means displays the corrected ultrasonic image on the screen.

2.    The ultrasonic diagnostic apparatus according to claim 1, **characterized by** further comprising storage means for storing volume image data other than an ultrasonics image captured by an image diagnostic apparatus in advance and reference image generation means for extracting tomogram image data corresponding to the ultrasonic image from the volume image data stored in the storage means and reconstructing a reference image, wherein
     the display means displays the corrected ultrasonic image on a same screen as the reference image.

3.    The ultrasonic diagnostic apparatus according to claim 1 or 2, **characterized in that**
     the strain calculation means obtains a strain distribution of a region-of-interest which is set in the ultrasonic image displayed on the display screen, and

the corrected ultrasonic image generation means corrects the ultrasonic image to remove strain in the region-of-interest on the basis of the strain distribution obtained by the strain calculation means and generates the corrected ultrasonic image.

4. The ultrasonic diagnostic apparatus according to claim 3, **characterized by** further comprising pressure measurement means for measuring a pressure which is applied to a body surface part of the object by the ultrasonic probe and pressure calculation means for obtaining a distribution of pressure acting on a body site in the region-of-interest on the basis of a pressure measurement value obtained by measurement by the pressure measurement means, wherein

the corrected ultrasonic image generation means includes enlargement ratio calculation means for obtaining a modulus of elasticity distribution of the body site in the region-of-interest on the basis of the pressure distribution in the region-of-interest calculated by the pressure calculation means and the strain distribution in the region-of-interest and obtaining an enlargement ratio distribution for removing strain in the body site in the region-af-interest in a pressed state and performing enlargement correction on the ultrasonic image on the basis of the obtained modulus of elasticity distribution and enlargement processing means for performing enlargement correction on the ultrasonic image in the pressed state on the basis of the enlargement ratio distribution obtained by the enlargement ratio calculation means and generating the corrected ultrasonic image in a non-pressed state.

5. The ultrasonic diagnostic apparatus according to claim 4, **characterized in that**
the enlargement ratio calculation means divides the region-of-interest into a plurality of microregions in a grid pattern, obtains a modulus of elasticity of each microregion on the basis of the pressure distribution and the strain distribution in the pressed state, and obtains an enlargement ratio for removing strain in each microregion on the basis of the modulus of elasticity of the microregion, and
the enlargement processing means performs enlargement correction on each microregion in the pressed state on the basis of the enlargement ratio obtained by the enlargement ratio calculation means and generates the corrected ultrasonic image.

6. The ultrasonic diagnostic apparatus according to claim 5, **characterized in that**
the strain calculation means obtains the strain distribution only in a depth direction of the region-of-interest, and the enlargement ratio calculation means obtains the modulus of elasticity distribution only in the depth direction of the region-of-interest and obtains the enlargement ratio distribution only in the depth direction of the region-of-interest.

7. The ultrasonic diagnostic apparatus according to claim 2, **characterized in that**
the display means displays the corrected ultrasonic image and the reference image side by side or such that the images are superimposed on each other.

8. An ultrasonic diagnostic apparatus **characterized by** comprising an ultrasonic probe which is pressed against a body surface of an object and transmits and receives an ultrasonic wave to and from the object, ultrasonic image generation means for forming an ultrasonic image on a scan plane of the ultrasonic probe on the basis of RF signal frame data of a reflected echo signal received via the ultrasonic probe, storage means for storing volume image data other than an ultrasonic image captured by an image diagnostic apparatus in advance, reference image generation means for extracting tomogram image data corresponding to the ultrasonic image from the volume image data stored in the storage means and reconstructing a reference image, and display means for displaying the ultrasonic image and the reference image on a same screen,
wherein strain calculation means for obtaining a strain distribution of a body site on the scan plane when pressed by the ultrasonic probe, on the basis of a pair of the RF signal frame data which are obtained at different measurement times and
corrected reference image generation means for correcting the reference image on the basis of the strain distribution obtained by the strain calculation means and generating a corrected reference image and the corrected reference image with strain are provided, and
the display means displays the corrected ultrasonic image on a same screen.

9. The ultrasonic diagnostic apparatus according to claim 8, **characterized in that**
the strain calculation means obtains a strain distribution of a region-of-interest which is set in the ultrasonic image displayed on the display screen, and
the corrected reference image generation means performs reduction processing on the reference image in the region-of-interest on the basis of the strain distribution obtained by the strain calculation means and generates the corrected reference image.

**10.** The ultrasonic diagnostic apparatus according to claim 8, **characterized in that**
the strain calculation means obtains a strain distribution of a region-of-interest which is set in the ultrasonic image displayed on the display screen,
the apparatus further comprises pressure measurement means for measuring a pressure which is applied to a body surface part of the object by the ultrasonic probe and pressure calculation means for obtaining a distribution of pressure acting on a body site in the region-of-interest on the basis of a pressure measurement value obtained by measurement by the pressure measurement means, and
the corrected reference image generation means includes reduction ratio calculation means for obtaining a modulus of elasticity distribution of the body site in the region-of-interest on the basis of the pressure distribution in the region-of-interest calculated by the pressure calculation means and the strain distribution in the region-of-interest and obtaining a reduction ratio distribution for correcting the reference image in the region-of-interest on the basis of the obtained modulus of elasticity distribution and reduction processing means for performing reduction correction on the reference image on the basis of the reduction ratio distribution obtained by the reduction ratio calculation means and generating the corrected reference image.

**11.** The ultrasonic diagnostic apparatus according to claim 10, **characterized in that**
the reduction ratio calculation means divides the region-of-interest into a plurality of microregions in a grid pattern, obtains a modulus of elasticity of each microregion on the basis of the pressure distribution and the strain distribution in the pressed state, and obtains a reduction ratio for adding strain in each microregion to the reference image on the basis of the modulus of elasticity of the microregion, and
the reduction processing means performs reduction correction on a microregion of the reference image corresponding to each microregion on the basis of the reduction ratio obtained by the reduction ratio calculation means and generates the corrected reference image.

**12.** The ultrasonic diagnostic apparatus according to claim 10, **characterized in that**
the reduction ratio calculation means obtains the reduction ratio distribution on a pixel-by-pixel basis of the region-of-interest, and
the reduction processing means performs reduction correction on the reference image corresponding to the region-of-interest pixel by pixel on the basis of the reduction ratio distribution obtained by the reduction ratio calculation means and generates the corrected reference image.

**13.** The ultrasonic diagnostic apparatus according to claim 10, **characterized in that**
the reduction ratio calculation means obtains the reduction ratio distribution on a pixel-by-pixel basis of the region-of-interest, and
the reduction processing means performs reduction correction on the reference image pixel by pixel on the basis of a reduction ratio or reduction ratios of one or adjacent ones of pixels in a depth direction of the reference image corresponding to the region-of-interest and generates the corrected reference image.

**14.** The ultrasonic diagnostic apparatus according to claim 13, **characterized in that**
the reduction processing means combines pieces of luminance information of the adjacent ones of the pixels into a piece of luminance information for one pixel.

**15.** The ultrasonic diagnostic apparatus according to claim 8, **characterized in that**
the display means displays the ultrasonic image and the corrected reference image on a same screen side by side or such that the images are superimposed on each other.

FIG.1

FIG.2(A)

FIG.2(B)

EP 2 123 224 A1

FIG.3(A)　　　　　　　　　FIG.3(B)　　　　　　　　　FIG.3(C)

LINE DIRECTION
X

| | 1 | 2 | 3 | 4 | 5 | 6 | 7 |
|---|---|---|---|---|---|---|---|
| 1 | 1.0 | 1.0 | 1.0 | 1.0 | 1.0 | 1.0 | 1.0 |
| 2 | 1.0 | 1.0 | 1.0 | 1.0 | 1.0 | 1.0 | 1.0 |
| 3 | 1.0 | 1.0 | 1.5 | 1.5 | 1.0 | 1.0 | 1.0 |
| 4 | 1.0 | 1.4 | 1.5 | 1.5 | 1.5 | 1.5 | 1.0 |
| 5 | 1.0 | 1.6 | 1.0 | 1.0 | 1.5 | 1.5 | 1.0 |
| 6 | 1.0 | 1.0 | 1.0 | 1.0 | 1.0 | 1.0 | 1.0 |
| 7 | 1.0 | 1.0 | 1.0 | 1.0 | 1.0 | 1.0 | 1.0 |
| 8 | 1.0 | 2.0 | 1.0 | 1.0 | 1.5 | 1.5 | 1.0 |
| 9 | 1.0 | 1.0 | 1.0 | 1.0 | 1.0 | 1.0 | 1.0 |

Y
DEPTH DIRECTION
ENLARGEMENT RATIO
Ai,j

MFD

LINE DIRECTION
X

Y
DEPTH DIRECTION

UFD

LINE DIRECTION
X

Y
DEPTH DIRECTION

DFD

EP 2 123 224 A1

FIG.4

```
              ┌──────────────┐
              │    START     │  STEP S1
              └──────┬───────┘
                     │
         ┌───────────▼────────────┐
         │  PERFORM INITIALIZATION │
         │         x←1             │
         └───────────┬────────────┘
                     │        STEP S2
                     ▼
              ╱───────────╲          False    ┌───────────────────────┐
             ╱    x≦N       ╲──────────────────│ PERFORM INITIALIZATION │  STEP S5
             ╲             ╱                   │         x←1            │
              ╲───────────╱                    └───────────┬───────────┘
   STEP S3         │True                                   │      STEP S6
      ┌────────────▼────────────┐                          ▼
      │ OBTAIN ORIGIN DEPTH Yo(x)│                   ╱───────────╲     False
      │ FOR ENLARGEMENT Yo(x)←M  │                  ╱    x≦N      ╲──────────┐
      └────────────┬────────────┘                  ╲            ╱           │
   STEP S4         │                                ╲──────────╱      ┌─────▼────┐
      ┌────────────▼────────────┐                       │True         │   END    │
      │   ADVANCE LINE BY 1      │              STEP S7  ▼             └──────────┘
      │        X←x+1            │       ┌────────────────────────────────┐
      └─────────────────────────┘       │ SHIFT TO ENLARGEMENT ORIGIN Yo(x)│
                                         │  y←Yo(x)  y2←Yo(x)  y3←Yo(x)     │
                                         └────────────────┬─────────────────┘
                                                          ▼
                                              ┌──────────────────┐
                                              │     Y3←y3+1      │  STEP S8
                                              └────────┬─────────┘
                                                       ▼
                                                ╱───────────╲   STEP S9
                                               ╱    y≧1      ╲
                                               ╲            ╱
                                                ╲──────────╱
                          STEP S15    False          │      True     STEP S10
                        ┌──────────────────┐         │      ┌──────────────────────┐
                        │ ADVANCE LINE BY 1 │◀────────┴──────│ OBTAIN DEPTH AFTER    │
                        │      x←x+1        │                │ ENLARGEMENT           │
                        └──────────────────┘                │   y3←ya-A(x,y)        │
                                                            └──────────┬───────────┘
                                                                       ▼   STEP S11
                                                                ╱───────────╲   False
                                                               ╱    Y2≧y3    ╲──────┐
                                                               ╲            ╱       │
                                                                ╲──────────╱        │
                                                                     │True          │
                                                    ┌────────────────▼─────────────┐│
                                          STEP S12  │ TRANSFER LUMINANCE           ││
                                                    │ INFORMATION TO OUTPUT IMAGE   ││
                                                    │      C(x,y2)←B(x,y)          ││
                                                    └────────────────┬─────────────┘│
                                                    ┌────────────────▼─────────────┐│
                                          STEP S13  │ DECREMENT DEPTH BY 1         ││
                                                    │      y2←y2-1                 ││
                                                    └────────────────┬─────────────┘│
                                                                     └──────────────┘
                                                                       │
                                                    ┌──────────────────▼───────────┐
                                          STEP S14  │  DECREMENT DEPTH BY 1        │
                                                    │       y←y-1                  │
                                                    └──────────────────────────────┘
```

22

FIG.5

OSP

CMP

STRAIN CORRECTION
RANGE
ROI

USP

RFP

STRAIN
CORRECTION RANGE
SPECIFICATION BUTTON

SST

EP 2 123 224 A1

FIG. 6

EP 2 123 224 A1

FIG.7(A)

FIG.7(B)

FIG.8(A)　　　　　FIG.8(B)　　　　　FIG.8(C)

Reduction ratio table (FIG.8(A)):

LINE DIRECTION X

| | 1 | 2 | 3 | 4 | 5 | 6 | 7 |
|---|---|---|---|---|---|---|---|
| 1 | 1.0 | 1.0 | 1.0 | 1.0 | 1.0 | 1.0 | 1.0 |
| 2 | 1.0 | 1.0 | 1.0 | 1.0 | 1.0 | 1.0 | 1.0 |
| 3 | 1.0 | 1.0 | 1.0 | 1.0 | 1.0 | 1.0 | 1.0 |
| 4 | 1.0 | 1.0 | 0.5 | 0.5 | 0.5 | 0.5 | 1.0 |
| 5 | 1.0 | 1.0 | 0.5 | 0.5 | 1.0 | 1.0 | 1.0 |
| 6 | 1.0 | 1.0 | 1.0 | 1.0 | 1.0 | 1.0 | 1.0 |
| 7 | 1.0 | 1.0 | 1.0 | 1.0 | 0.8 | 0.8 | 1.0 |
| 8 | 1.0 | 1.0 | 1.0 | 1.0 | 0.8 | 0.8 | 1.0 |
| 9 | 1.0 | 1.0 | 1.0 | 1.0 | 1.0 | 1.0 | 1.0 |

Y DEPTH DIRECTION

REDUCTION RATIO Ri,j

MFD

RFD

OFD

## INTERNATIONAL SEARCH REPORT

| International application No. |
|---|
| PCT/JP2007/074550 |

**A. CLASSIFICATION OF SUBJECT MATTER**
*A61B8/08*(2006.01)i

According to International Patent Classification (IPC) or to both national classification and IPC

**B. FIELDS SEARCHED**

Minimum documentation searched (classification system followed by classification symbols)
A61B8/08

Documentation searched other than minimum documentation to the extent that such documents are included in the fields searched
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2005-102713 A (Hitachi Medical Corp.), 21 April, 2005 (21.04.05), Par. No. [0019] (Family: none) | 1-15 |
| A | H. GholamHosseini et al, Fusion of Vibro-acoustography Images and X-ray Mammography, Proceedings of the 28th IEEE EMBS Annual International Conference, 2006.08.30, pp.2803-2806 | 1-15 |

☒ Further documents are listed in the continuation of Box C.　　☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19 March, 2008 (19.03.08) | 01 April, 2008 (01.04.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

27

**INTERNATIONAL SEARCH REPORT**

International application No.

PCT/JP2007/074550

C (Continuation).   DOCUMENTS CONSIDERED TO BE RELEVANT

| Category* | Citation of document, with indication, where appropriate, of the relevant passages | Relevant to claim No. |
|---|---|---|
| A | Microfilm of the specification and drawings annexed to the request of Japanese Utility Model Application No. 26738/1983(Laid-open No. 76910/1985) (Hitachi Medical Corp.), 29 May, 1985 (29.05.85), Description, page 8, lines 7 to 16; Fig. 1 (Family: none) | 1-15 |
| A | JP 2006-20746 A  (Hitachi Medical Corp.), 26 January, 2006 (26.01.06), Abstract (Family: none) | 1-15 |

Form PCT/ISA/210 (continuation of second sheet) (April 2007)

28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004098414 A1